**Europäisches Patentamt**

**European Patent Office**

**Office européen des brevets**

(19)

(11) Numéro de publication: **0 065 924**
**A1**

# (12) DEMANDE DE BREVET EUROPEEN

(21) Numéro de dépôt: **82400941.9**

(22) Date de dépôt: **19.05.82**

(51) Int. Cl.³: **C 12 N 15/00**
**C 07 H 21/04, C 12 N 1/20**
**C 12 P 21/02**
**//C12R1/19, A61K39/13**

(30) Priorité: **19.05.81 FR 8109968**

(43) Date de publication de la demande:
**01.12.82 Bulletin 82/48**

(84) Etats contractants désignés:
**IT**

(71) Demandeur: **INSTITUT PASTEUR**
**25-28, rue du Docteur Roux**
**F-75724 Paris Cedex 15(FR)**

(72) Inventeur: **Girard, Marc**
**6, rue César Franck**
**F-75015 Paris(FR)**

(72) Inventeur: **Kourilsky, Philippe**
**207, rue de Vaugirard**
**F-75015 Paris(FR)**

(72) Inventeur: **Kopecka, Helena**
**13, rue Léon Bloy**
**F-92260 Fontenay Aux Roses(FR)**

(72) Inventeur: **Van der Werf, Sylvie**
**3, Villa Grenelle**
**F-75015 Paris(FR)**

(72) Inventeur: **Bregegere, François**
**Tour Verdi-Apt.41-99 19, rue de Choisy**
**F-75643 Paris Cedex 13(FR)**

(74) Mandataire: **Gutmann, Ernest et al,**
**Cabinet Plasseraud 84, rue d'Amsterdam**
**F-75009 Paris(FR)**

(54) **Fragments d'ADN portant des parties de l'information génétique des poliovirus, notamment du type PV1, et procédé pour leur obtention.**

(57) L'invention concerne un fragment clonable originaire du génome d'un poliovirus. Il est formé d'un segment hybridable à une partie du poliovirus du type PV-1, ce segment codant pour les protéines de capside et pour les éventuelles protéases nécessaires au découpage de celles-ci, cependant essentiellement à l'exclusion d'un fragment hybridable avec la partie terminale du poliovirus, comportant au moins 1,7 à 1,8 kilobases à partir de son extrémité terminale 3' et codant pour les protéines intervenant dans la réplication du poliovirus.

EP 0 065 924 A1

Fragments d'ADN portant des parties de l'information
génétique des poliovirus, notamment du type PV1, et
procédé pour leur obtention.

———————

L'invention est relative à des fragments d'ADN
ayant, au niveau de l'information génétique qu'ils contiennent, des parties en commun avec les poliovirus, de
préférence mais non exclusivement du type PV-1. En particulier, l'invention est relative à des ADN codant pour
les protéines de capside et pour les éventuelles protéases nécessaires au découpage de celles-ci, cependant
essentiellement à l'exclusion d'un fragment hybridable
avec la partie terminale du poliovirus codant pour les
protéines intervenant dans la réplication du poliovirus.
Plus particulièrement encore, l'invention concerne de
tels ADN, qui comportent également les nucléotides de
l'extrémité 5' du poliovirus du type PV-1 (ou PV1).

Bien qu'ayant déjà fait l'objet de nombreuses
études, la structure du génome du poliovirus PV-1 n'est
encore que très partiellement connue. Quelques sites de
restriction par des enzymes spécifiques ont déjà été
décrits et localisés, mais la méconnaissance de la
carte de la majeure partie du génome a rendu impossible
à ce jour l'étude et la construction de fragments de
longueur déterminée et correspondant à des parties déterminées de ce génome.

L'ARN a cependant fait l'objet d'études poussées.
Les informations qui en ont été tirées contribuent certes
à une meilleure connaissance du génome lui-même du poliovirus. Il a été possible grâce à la digestion de cet ARN
par une RNase T1 (ou nucléase T1) d'isoler un certain
nombre d'oligonucléotides résistant à la RNase T1, de les
caractériser (référence 6) et même de déterminer les emplacements relatifs des séquences nucléotidiques du génome auxquelles correspondent lesdits oligonucléotides (21). Ces oligonucléotides seront dans ce qui suit désignés par les mêmes numéros que ceux utilisés par Y.F. LEE (dans la publication 6).

L'invention a pour but de remédier aux difficultés susmentionnées, notamment en ce qui concerne la production de fragments d'ADN de longueur déterminée, plus particulièrement de ceux portant l'information nécessaire pour diriger la synthèse des propriétés antigéniques, de préférence aussi immunogéniques, spécifiques au poliovirus, cependant en l'absence des parties du génome de ces derniers qui portent les gènes leur conférant un caractère infectieux.

L'invention a également plus particulièrement pour but la production de tels fragments, ceux-ci comprenant cependant la totalité des nucléotides spécifiques de la région d'extrémité 5' du poliovirus.

L'invention découle de la découverte que le clonage dans une bactérie d'un hybride de cADN-ARN avait abouti en fait à la production d'une multitude de clones contenant des fragments de différentes longueurs et correspondant respectivement à des régions différentes du cADN entier susceptible d'être synthétisé à partir de l'ARN du poliovirus PV-1. De plus l'invention tire profit de la combinaison qui a été réalisée des techniques d'analyse de ces divers fragments par des enzymes de restriction, d'une part, et de la technique d'hybridation *in situ* qui peut être observée entre lesdits fragments et divers nucléotides résistant à la RNase, d'autre part, pour établir :

- les cartes de restriction d'un certain nombre des fragments de cADN clonés,
- les positions relatives que devaient avoir ces mêmes fragments d'ADN vis-à-vis du cADN entier correspondant au génome du poliovirus PV-1 et, par conséquent, la carte de restriction correspondante du génome entier du poliovirus PV-1.

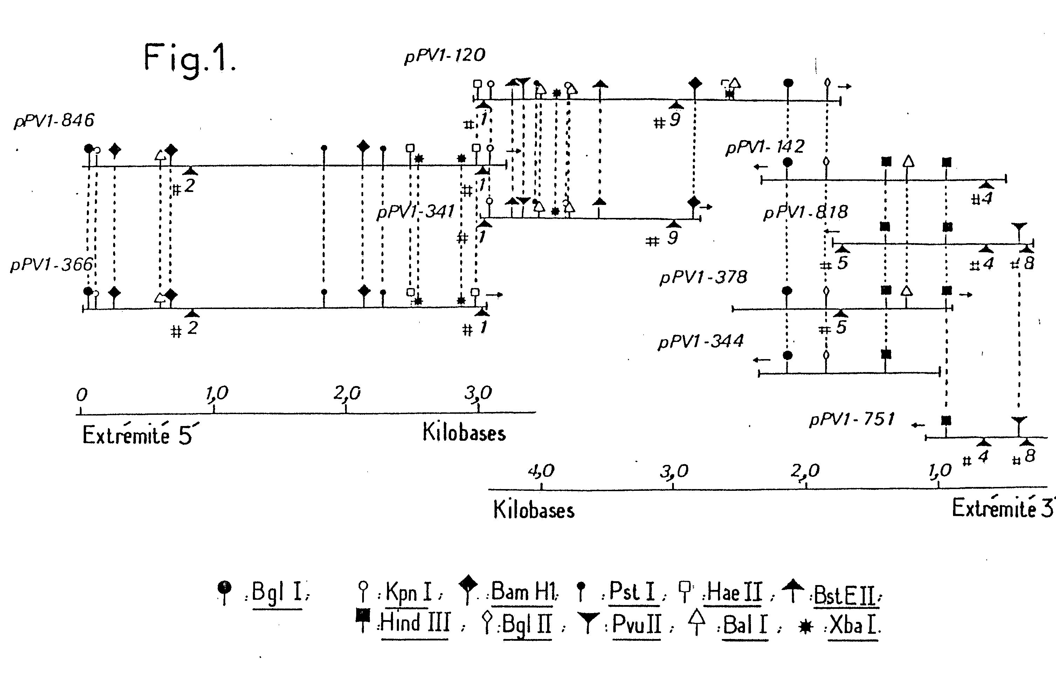

En effet, on constate qu'après clonage aucun des clones n'est en général capable de s'hybrider à tous les nucléotides résistant à la nucléase T1, que néanmoins la plupart des clones conduisent à des hybridations avec un seul oligonucléotide, voire avec quelques oligonucléotides résistant à la RNase T1 et connus pour s'hybrider avec des zones voisines ou adjacentes du génome entier du poliovirus. Il en est résulté la possibilité de préciser les positions relatives des fragments cADN clonés vis-à-vis du génome entier, comme cela ressort de la figure 1. Les analyses de restriction opérées sur les différents fragments ont permis de déterminer leurs posi- tions relatives entre eux de façon beaucoup plus précise, par là mise en alignement des sites de restriction contenus dans leurs parties communes.

La réalisation d'une carte de restriction du génome entier du poliovirus sera explicitée plus loin à l'occasion de la description de l'ensemble des essais qui ont été réalisés pour aboutir à l'établissement de la carte de restriction globale.

La fragmentation du cADN (résultant de la "réparation" par les cellules transformées elles-mêmes des fragments correspondants du cADN-ARN hybride) au cours de l'opération de clonage initial était d'autant plus inattendue que la synthèse du cADN avait été amorcée par l'oligo(dT) au niveau du poly.(A) terminal 3' de l'ARN viral, de sorte que l'on s'attendait à l'obtention de cADN cloné ayant aussi une extrémité intacte 3'. Mais ce résultat inattendu a été transformé en un avantage, puisqu'il a permis, à l'aide de la combinaison des techniques évoquées ci-dessus, de réaliser la distribution des différents fragments d'ADN clonés obtenus, sur toute la longueur du cADN correspondant, par conséquent du génome lui- même.

Au titre des résultats inattendus, il faut également noter l'isolement de certains clones dont les fragments de ces ADN d'insertion se sont révélés contenir la séquence d'extrémité 5' entière du poliovirus PV-1. Un tel résultat ne pouvait pas être obtenu par le clonage en lieu et place de l'hybride cADN-ARN, du cADN lui-même correspondant au génome entier du virus, en raison de la formation bien connue d'une boucle de nucléotides altérant l'extrémité 5', au cours de la formation du second brin de ce cADN, sous l'action de la polymérase appropriée.

L'ADN selon l'invention est caractérisé en ce qu'il consiste ou est formé d'un segment hybridable à une partie du poliovirus du type PV-1, ce segment codant pour les protéines de capside et pour les éventuelles protéases nécessaires au découpage de celles-ci, cependant essentiellement à l'exclusion d'un fragment hybridable avec la partie terminale du poliovirus, comportant au moins 1,7 à 1,8 kilobases à partir de son extrémité terminale 3' et codant pour les protéines intervenant dans la réplication du poliovirus.

Le procédé selon l'invention pour obtenir un tel ADN est alors caractérisé en ce que l'on réalise une recombinaison génétique entre les fragments de cADN, tels qu'ils ont été obtenus ci-dessus, après leur analyse dans les conditions déjà évoquées, notamment par le recours aux enzymes de restriction appropriés auxquels correspondent des sites spécifiques dans les régions que les divers fragments ont en commun, notamment selon la méthodologie qui sera donnée plus loin à propos de la réalisation d'un exemple.

Conformément à l'une des caractéristiques préférées de l'invention, l'ADN sus-défini comprend, du côté de son extrémité 5', les nucléotides d'extrémité du poliovirus du type PV-1, plus particulièrement la séquence

```
                  10         20         30         40
   (G)₂₀ TTAAAACAGC TCTGGGGTTG TACCCACCCC AGAGGCCCAC
                  50         60         70         80
          GTGGCGGCTA GTACTCCGGT ATTGCGGTAC CCTTGTACGC
                  90        100
          CTGTTTTATA CTCCCTTCCC
```

En conformité avec les résultats des analyses de restriction dans les conditions qui seront indiquées plus loin, l'ADN selon l'invention peut également être défini par référence à la série des sites de restriction

successifs qu'il contient, dont les positions peuvent alors être déterminées, par exemple vis-à-vis d'un site BamHI, lui-même situé à une distance de l'ordre de 0,7 kilopaires de bases (kb ou kbp selon les abréviations utilisées plus loin) de l'extrémité 5' du génome du poliovirus. Ces positions sont alors les suivantes (distances exprimées en kb et affectées d'un signe "-", pour ceux des sites situés entre ladite extrémité 5' du génome du poliovirus et du site BamHI considéré) :

| | | | | | |
|---|---|---|---|---|---|
| Pst I | : 1,1 | ; 1,55 | ; 2,75 | | |
| Pvu II | : 2,6 | | | | |
| Kpn I | : -0,63 | ; 2,77 | ; 3,37 | | |
| Bgl II | : 4,9 | | | | |
| Bam HI | : -0,45 | ; 1,4 | ; 3,9 | | |
| Bal I | : -0,05 | ; 2,80 | ; 3 | ; 4,25 | |
| Bst EII | : 2,55 | ; 3,20 | | | |
| Bgl I | : -0,66 | ; 4,56 | | | |
| Bcl I | : 2,50 | ; 3,25 | | | |

Il va de soi que certains de ces sites pourront disparaître à l'occasion de délétions qui n'affecteraient pas le caractère antigénique ou immunogénique des produits d'expression susceptibles d'être obtenus au terme de la transformation de cellules appropriées par des vecteurs contenant de tels fragments d'insertion.

Dans la mesure où il semble établi que la région d'initiation de la traduction du génome du poliovirus PV-1 est située à une distance de l'ordre de 741 paires de bases de l'extrémité 5' du génome, on peut envisager de renoncer dans les fragments selon l'invention à la région correspondante, cas dans lequel l'ADN tel qu'il a été défini ci-dessus pourrait être modifié par délétion de la région d'extrémité 5' correspondante. Par exemple l'ADN selon l'invention peut alors être caractérisé par une extrémité 5' formée soit par le site Bam HI de référence, lorsque toute la partie antérieure a été délétée, soit par l'extrémité Bal I (-0,05 kb), ou Kpn I (-0,63 kb) ou Bgl I (-0,66 kb), notamment en cas de délétions partielles de ladite partie antérieure.

6

Avantageusement, le fragment d'ADN selon l'invention est limité du côté de son extrémité 3', soit par un site Bgl II (4,9 kb), soit par une extrémité Bgl I (4,56 kb), soit Bal I (4,25 kb), soit Bam HI (3,9 kb). On peut encore également caractériser les ADN selon l'invention par leurs capacités d'hybridation avec les sondes d'oligonucléotides dérivés de l'ARN du poliovirus PV-1 et résistant à la nucléase T1 et comportant des extrémités nucléotidiques 5', dont les positions respectives dans la séquence nucléotidique totale de l'ARN du poliovirus (repérées en nombres (N) de paires de bases comptées à partir de sa propre extrémité 5') sont les suivantes :

N806  (oligonucléotide n° 2)

N3000 (oligonucléotide n° 1)

N4434 (oligonucléotide n° 9).

Ces derniers ADN selon l'invention peuvent être obtenus par des variantes du procédé défini ci-dessus consistant à cloner un cADN résultant soit de l'hydrolyse totale du cADN entier correspondant au génome du poliovirus PV-1 par l'enzyme de restriction Bgl I ou Bgl II, soit de l'hydrolyse partielle par l'enzyme de restriction Bal I ou Bam I, pour isoler les fragments ayant les longueurs voulues.

Il n'est pas sans intérêt de noter ici que, dans la mesure où l'on recherche un cADN final conforme à l'invention comprenant l'extrémité 5' intacte du génome de PV-I, on peut également cloner le cADN résultant de la recombinaison génétique de l'un des cADN parmi ceux produits à l'issue du clonage de l'hybride PV-1 cADN-ARN et qui comporte la région d'extrémité 5' de PV1, d'une part, avec le cADN entier qui précède, d'autre part, après traitement de ces cADN avec l'une des enzymes de restriction auxquelles correspondent des sites dans la région commune à ces deux cADN.

Le cADN final obtenu peut alors à son tour être incorporé dans un vecteur, par exemple pBR322, dans les conditions qui sont décrites plus loin ou des conditions analogues, le vecteur entier étant ensuite utilisé pour transformer des cellules hôte appropriées dans lesquelles le fragment en question est susceptible d'être exprimé.

L'invention concerne donc également l'application de ces ADN à la production de principes antigéniques comprenant la transformation avec cet ADN d'une cellule hôte dans laquelle il est susceptible d'être exprimé, le cas échéant après incorporation préalable de cet ADN dans un vecteur approprié, contenant un promoteur susceptible d'initier la traduction de cet ADN dans ladite cellule hôte, et la récupération à partir des cellules transformées ou de leur milieu de culture, selon le cas, des principes antigéniques produits.

On peut à cet égard avoir recours à des techniques désormais classiques.

Des caractéristiques complémentaires de l'invention apparaîtront encore au cours de la description plus détaillée qui suit de la mise en oeuvre des différents aspects de l'invention, notamment avec référence aux dessins, dans lesquels les figures font apparaître les faits suivants :

La figure 1 fournit une représentation des cartes de restriction de plusieurs des fragments de cADN qui ont été obtenus après transformation de *E. coli* par un cADN-ARN hybride du genre en question, lesdits fragments d'ADN étant placés dans les positions relatives permettant d'amener en alignement (ou en coïncidence) les régions qui leur sont communes, par conséquent les sites de restriction communs et les emplacements dans lesquels ils présentent des hybridations *in situ* avec certains des oligonucléotides numérotés résistant à la RNase T1 ci-dessus visés (les numéros correspondant figurant sous une tête de flèche et étant précédés par le signe "#"). Les signes utilisés pour repérer les régions spécifiques aux sites de diverses enzymes de restriction sont également définis dans la figure 1.

Les petites flèches correspondent à l'orientation des fragments d'insertion correspondants vis-à-vis du vecteur porteur pBR322.

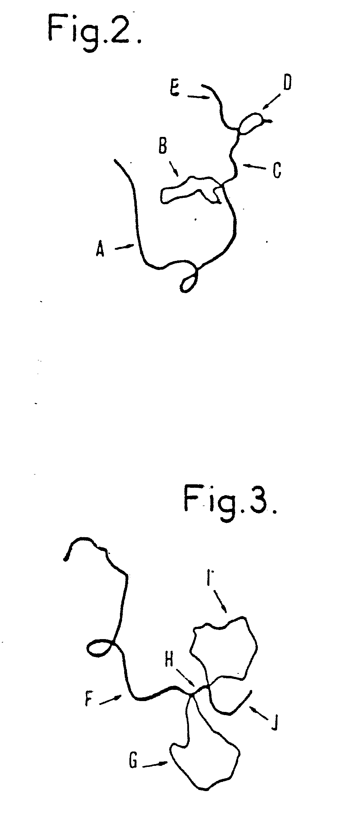

La figure 2 et la figure 3 sont des représentations de molécules hétéroduplex formées entre des fragments délimités par des extrémités EcoRI, obtenues par ouverture des plasmides contenant des fragments d'insertion présentant eux-mêmes des séquences nucléotidiques communes.

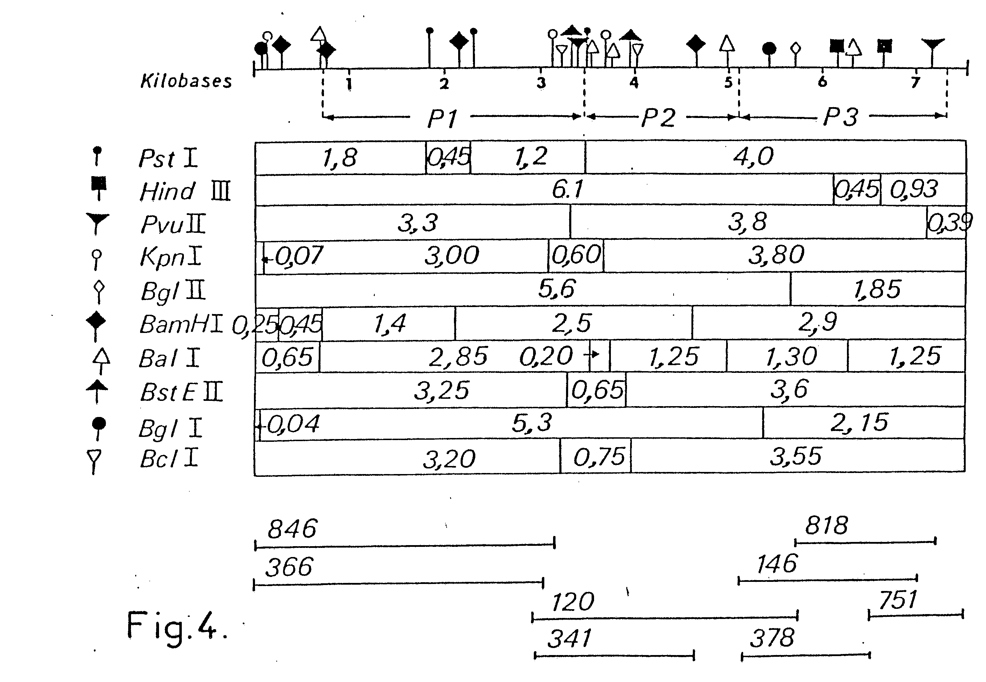

La figure 4 représente la carte de restriction obtenue pour l'ensemble du génome du poliovirus. Les positions des différents sites de restriction y sont présentées. Y figurent également de façon schématique les positions relatives vis-à-vis du génome entier des fragments d'insertion de ceux des clones qui ont été retenus pour établir la carte de restriction du genre en question.

Les longueurs des différents fragments qui séparent, soit deux sites identiques entre eux, soit un site unique, lorsque tel est le cas des extrémités respectivement 5' et 3' du génome entier du poliovirus, y sont exprimées en nombre de kilobases.

Les figures 5a à 5h sont une représentation schématique des différentes opérations qui ont été effectuées pour produire un cADN conforme à l'invention.

Construction et criblage ("screening") de clones contenant des ADN recombinants :

Une souche de poliovirus Mahoney appartenant au sérotype PV-1 est développée dans des cultures de cellules HEla en suspension et on extrait l'ARN viral selon la technique décrite par LEE et Coll. (référence 6). Un cDNA est préparé à partir de ce RNA en mettant en oeuvre une amorce constituée par un oligo(dT)$_{10}$ et une transcriptase réverse selon la méthode décrite par KITAMURA et Coll. (référence 7). Les molécules hybrides cDNA-RNA obtenues sont purifiées par centrifugation dans un gradient de sucrose neutre 15-30 %, les extrémités de ces cDNA-RNA hybrides étant ensuite rognées avec de la RNase A (25 microgrammes/millilitre) et de la RNase T1 (0,05 U/millilitre) en présence de NaCl 0,3 M. On allonge les extrémités 3' des molécules obtenues par des fragments poly (dC)sous la direction d'une transférase terminale. De même on allonge les extrémités 5' d'un vecteur de clonage pBR322 préalablement ouvert au niveau de son site PstI au moyen de poly(dG), notamment selon la technique décrite par NELSON et Coll. (référence 8). Le DNA de pBR322 ainsi modifié (450 ng) et des molécules de cDNA-RNA comportant les allongements terminaux de poly(dC) (300 ng) sont réunis par hybridation, notamment par les techniques décrites par WOOD et Coll. (référence 9) et ZAIN et Coll. (référence 10), et utilisés pour transformer *E.coli* 1106 803r$_k$-m$_k$- (MURRAY et Coll., référence 11) (C.N.C.M. le 19 mai 1981 sous le n° I-157).

Les clones bactériens contenant des ADN recombinants sont sélectionnés sur la base de leur résistance à la tétracycline et leur sensibilité à l'ampicilline. 800 colonies ont ainsi été isolées.

Analyse des cDNAs clonés à l'aide d'enzymes de restriction:

Les plasmides contenus dans les clones sus-indiqués sont isolés et les cDNA contenus dans ces plasmides sont extraits par la technique des lysats clairs selon CLEWEL et Coll. (référence 16). Les cDNA sont séparés par centrifugation jusqu'à l'équilibre dans des gradients de CsCl/ bromure d'éthidium ou selon la méthode décrite par BIRNBOIM et Coll. (référence 17). Les cDNA peuvent être purifiés davantage encore par centrifugation dans un gradient de sucrose 5-40 %, notamment en vue de l'étude analytique en microscopie électronique. Les analyses au moyen d'endonucléases de restriction mettent en oeuvre des électrophorèses sur gel d'agarose ou de polyacrylamide dans un tampon TAE(Tris 40 mM,acétate 20 mM, pH 7,8, EDTA 2 mM). Les fragments sont visualisés par UV, après coloration par le bromure d'éthidium (2 μg/ml). Si nécessaire, on procède à une purification supplémentaire des fragments de restriction par électro-élution à partir du gel dans le tampon TAE dilué au 1/10 et à une chromatographie sur des colonnes d'hydroxyapatite, selon la technique décrite par MAY et Coll. (référence 18). On procède à la classification des fragments selon une carte linéaire par des doubles digestions appropriées.

Etude des positions relatives des fragments de cDNA clonés vis-à-vis du génome du poliovirus PV-1 :

Les clones ont été criblés ou triés au moyen d'une hybridation in situ , en ayant recours à des molécules courtes du cADN PV-1 à brin simple marquées radioactivement par du $^{32}$P, utilisées en tant que sonde spécifique de l'extrémité 3' du génome du poliovirus PV1. On a constaté qu'environ 10 % seulement des cellules transformées ont donné lieu à une hybridation positive.

Pour identifier les autres clones on a eu recours à la technique d'hybridation sur filtre des colonies de Grunstein-Hogness (références 13,14), en mettant en oeuvre, à titre de sondes d'hybridation, des oligonucléotides du type sus-défini, résistant à la nucléase -T1 et dont les extrémités avaient été marquées au $^{32}$P.

L'hybridation est effectuée pendant 60 heures à la température de 42°C, dans une solution dont la composition est la suivante : 1 x solution de Denhart (15) 2 x SSC (NaCl 0,30 M;citrate de sodium 0,030 M), EDTA 2 mM,$KH_2PO_4$ 25 mM (ajusté à PH 7,2 avec NaOH), SDS 0,5 % et ARN de transfert de levure (Sigma) 5 ug/ml. Les filtres ont été lavés 4 fois à 42°C dans une solution de 1 X Denhart, 2 x SSC, 0,5% SDS (chaque fois pendant une heure) puis 3 fois dans 2 x SSC (chaque fois 10 minutes). Les filtres ont été ensuite traités avec 2,5 µg/ml de RNase dans une solution de 2xSSC à 41°C pendant 45 minutes, lavés encore 3 fois dans 2xSSC, puis soumis à une autoradiographie.

On a également utilisé des sondes d'hybridation de cADN marquées au $^{32}$P, qui ont été préparées par incubation limitée d'ARN PV-1, avec une transcriptase réverse et des oligo (dT)10 en présence de(α $^{32}$P) ou de fragments de restriction dont les extrémités étaient marquées au $^{32}$P. Les conditions d'hybridation ont été celles décrites en (14).

L'analyse de la taille des fragments d'insertion originaires du cADN du poliovirus montre en outre qu'aucun n'avait une longueur supérieure à 3,2kb.

Les hybridations observées ont permis, comme exposé ci-après, de classer les clones, comme cela résulte du tableau I ci-après et de déterminer les positions relatives (montrées dans la figure 1) des cADN clonés vis à vis du génome initial.

De la figure 1 découlent également les natures et positions correspondantes des différents sites de restriction qui existent dans le cADN du poliovirus entier.

TABLEAU I

| Numéro du clone | HYBRIDATION AVEC | | | | | | | Taille des fragments d'insertion cADN insérés (kb) |
|---|---|---|---|---|---|---|---|---|
| | Oligonucléotide résistant à la RNase T1 | | | | | | Sonde cADN | |
| | = 2 (N 806) | = 1 (N 3000) | = 9 (N 4434) | = 5 (N 5693) | = 4 (N 6775) | = 8 (N 7125) | | |
| pPV1 - 378 | - | - | - | + | - | - | + | 1.65 |
| pPV1 - 344 | - | - | - | - | - | - | + | 1.45 |
| pPV1 - 142 | - | - | - | ND | + | - | + | 1.90 |
| pPV1 - 818 | - | - | - | + | + | + | + | 1.45 |
| pPV1 - 751 | - | - | - | - | + | + | + | 1.00 |
| pPV1 - 341 | - | ± | + | - | - | - | ND | 1.70 |
| pPV1 - 120 | - | + | + | ND | - | - | + | 2.80 |
| pPV1 - 366 | + | + | - | - | - | - | - | 3.05 |
| pPV1 - 846 | + | + | - | - | - | - | - | 3.20 |

Hybridation sur filtre des colonies avec des oligonucléotides-T1 dont les extrémités sont marquées au phosphore 32 ou avec une sonde cADN marquée au phosphore 32. Les oligonucléotides sont numérotés selon les indications fournies dans la publication sous référence 21 ; les numéros entre parenthèses (par exemple N 806) se réfèrent à la position du nucléotide 5'-terminal de l'oligonucléotide correspondant vis-à-vis de la séquence nucléotidique totale, comme calculé dans la direction 5'-3'. Les tailles des fragments d'insertion cADN ont été estimées grâce à leur mobilité dans l'agarose.

L'analyse des différents fragments d'insertion a conduit aux résultats suivants.

a) Extrémité_3'_du_génome_PV-1

Deux clones, pPV1-818 et pPV1-715 qui se sont hybridés avec la sonde spécifique du cADN de l'extrémité 3' du poliovirus se sont aussi hybridés avec les oligonucléotides-T1 N° 8 et 4, lesquels se situent eux-mêmes à l'intérieur de la séquence des 650 nucléotides qui s'étendent à partir de l'extrémité 3' de l'ARN correspondant du poliovirus (7).

Comme représenté à la figure 1 les cartes de restriction des fragments d'insertion correspondants ont été disposés de façon à ce que les sites de restriction PVuII et HindIII communs à ces deux fragments 818 et 715 soient en alignement. On a ainsi pu constater que les positions de ces deux sites coïncidaient avec les sites correspondants déjà connus pour ces deux enzymes dans le génome entier du poliovirus, c'est à dire à des distances correspondant à 389 et 926 paires de bases de l'extrémité 3' du poliovirus.

Grâce à l'établissement des cartes de restriction des cADN contenus dans les clones pPV1-142, pPV1-344 et pPV1-378, on a constaté l'existence d'un second site HindIII, situé à environ 0,45 kb en aval du précédent, et plus en aval encore de sites de restriction Bgl II et Bgl I séparés entre eux par environ 0,3 kb ( Fig. 1). Leur corrélation a été confirmée par la capacité qu'ont eu les deux fragments pPV1-378 et pPV1-818 de s'hybrider avec l'oligonucléotide N° 5.

pPV1-142 a en outre donné lieu à une hybridation avec l'oligonucléotide N° 4.

b) Milieu_du_génome

Les cartes de restriction des clones s'hybridant avec les oligonucléotides T1 connus pour s'hybrider avec le génome de PV-1 dans sa partie centrale (N°1 et N° 9) ont été placés de façon à amener en alignement les sites Bgl I et Bgl II, séparés d'environ 0,3 kb que tous deux possédaient également. Un fragment de 2,8 kb contenu dans pPV1-120 répondait aussi à ces conditions. Il s'est avéré contenir également des sites de clivage par des enzymes de restriction supplémentaires, dont la plupart étaient également dans le clone pPV1-341 (fig. 1).

Il est alors apparu que pPV1-120 devait avoir des parties communes avecpPV1-142 , pPV1-378 et pPV1-344; ce qui a été confirmé par l'étude des séquences présumées homologues,par analyse au microscope électronique de molécules hétéroduplex formées entre les produits de digestion par EcoRI contenant ces ADN. Les molécules hétéroduplex formées entre les ADN pPV1-120 et pPV1-378 se sont alors révélées présenter deux boucles D à brin simple (B et D) ayant des longueurs de 2,3 $\pm$ 0,35 et 1,16 $\pm$0,21kb respectivement (fig.2), séparées par une région centrale d'homologie (C) de 715 $\pm$ 75 paires de bases comme la carte de la figure 1 permettait de le prédire.

Le fait que le fragment de restriction Bgl I-Bgl II de 0,3 kb provenant de l'extrémité 3' de pPV1-120(fig.1) préalablement purifié, préalablement marqué à son extrémité au phosphore 32,s'hybridait <u>in situ</u> avec les clones <u>pPV1-344 et pPV1-378</u> a fourni une preuve supplémentaire du recouvrement de ces deux clones.

De même on a pu établir la présomption du recouvrement des fragments d'insertion 120, 378, 344 et 142 comme indiqué figure 1.

c) <u>Extrémité 5' du génome</u>

Plusieurs clones on pu être hybridés avec l'oligonucléotide-T1 n° 2 (Tableau 1) situé à environ 800 nucléotides à partir de l'extrémité 5' (21) mais aucun avec l'oligonucléotide de N° 21, qui se situe au 85 ème nucléotide à partir de l'extrémité 5' (23). Cependant on a pu formuler l'hypothèse que plusieurs clones chevauchaient la région du poliovirus en relation avec l'oligonucléotide N° 21, en raison de la présence dans ces clones de sites <u>Bgl I</u> et <u>Kpn I</u> dont les positions étaient compatibles avec les positions 35 et 66 qu'occupent les mêmes sites dans PV-1 à proximité de son extrémité 5'. Ces positions avaient elles-mêmes été déduites à partir de la séquence de nucléotides de l'extrémité 5' de l'ARN correspondant(23) . En particulier ces deux sites ont été trouvés dans les clones pPV1-846 et pPVI-366. L'analyse séquentielle des nucléotides de l'extrémité 5' de pPV1-366 a de plus conduit à la structure spécifique indiquée.

L'extrémité 5' de pPV1-366 comportait en outre un bout terminal de 20 résidus dG, suivi par 50 nucléotides dont la séquence est en accord parfait avec celle déterminée pour l'extrémité 5' de l'ARN (23,24) correspondant à PV1. Il en résultait que le fragment d'insertion du clone pPV1-366 commençait exactement au premier nucléotide du génome du poliovirus. La différence, voirel'impossibilité pour le nucléotide n° 21 de former un hybride stable avec pPV1-366 ou pPV1-846 dans le test d'hybridation, peut vraisemblablement être attribuée à la petite taille de la sondde.

Le fragment d'insertion 846 ayant une longueur de 3,2 kb s'est révélé contenir aussi un second site Kpn I situé à 3 kb du premier, et proche (environ 0,1 kb) d'un site Hae II. Puisque le fragment d'insertion 120 présentait aussi, à proximité de son extrémité 5',des sites Kpn I et Hae II séparés par environ 0,1 kb,on pouvait formuler l'hypothèse selon laquelle la région d'extrémité 3' du fragment d'insertion 846 et la région d'extrémité 5' du fragment d'insertion 120 présentaient la séquence commune de nucléotide schématisé à la figure 1.

Cette hypothèse a été renforcée par l'observation que les deux clones s'hybridaient à l'oligonucléotide n° 1,dont la localisation dans la région correspondante de l'ARN de PV1 est connue (21).

La configuration du chevauchement ou recouvrement entre pPV1-846 et pPV1-120 a été obtenue par l'examen au microcospe électronique des molécules hétéroduplex formées entre les fragments obtenus par digestion de ces plasmides en présence d'EcoRI.

Les molécules hétéroduplex (fig. 3) ont présenté deux boucles D à double brin (G et I), ayant des longueurs respectives de

$3,28 \pm 0,52$ et $2,82 \pm 0,4$, séparées par une région homologue centrale (H) de $195 \pm 50$ paires de bases, comme prévu d'après la carte de la figure 1. L'hybridation in situ avec le clone bactérien pPV1-846 du fragment d'extrémité 5' pst I - pst I de 0,45 kb, extrait de pV1-120 purifié et marqué au $^{32}$P, venait apporter une preuve supplémentaire du recouvrement.

3 - Reconstruction_d'une_carte_de_restriction_du_génome PV-1_entier

La carte de restriction du génome PV-1 pouvait en conséquence être reconstituée à partir de ceux des clones successifs donnant lieu à recouvrement mutuel, notamment des fragments d'insertion 846, 120, 142 et 751. La carte de restriction représentée fig. 4 du génome total du poliovirus PV1 découle par conséquent des analyses par les enzymes de restriction des susdits fragments d'insertion .

L'extrémité 5' du fragment 366 coïncide avec le nucléotide terminal 5' réel de PV1.

A l'inverse le fragment 751 coïncide avec la région d'extrémité 3' de PV1.

La taille de quelques fragments de restriction principaux et leurs emplacements relatifs vis à vis du génome entier sont données à la figure 4 avec une précision d'environ $\pm$ 200 paires de bases pour les plus longs, et $\pm$ 50 paires de bases pour les plus courts.

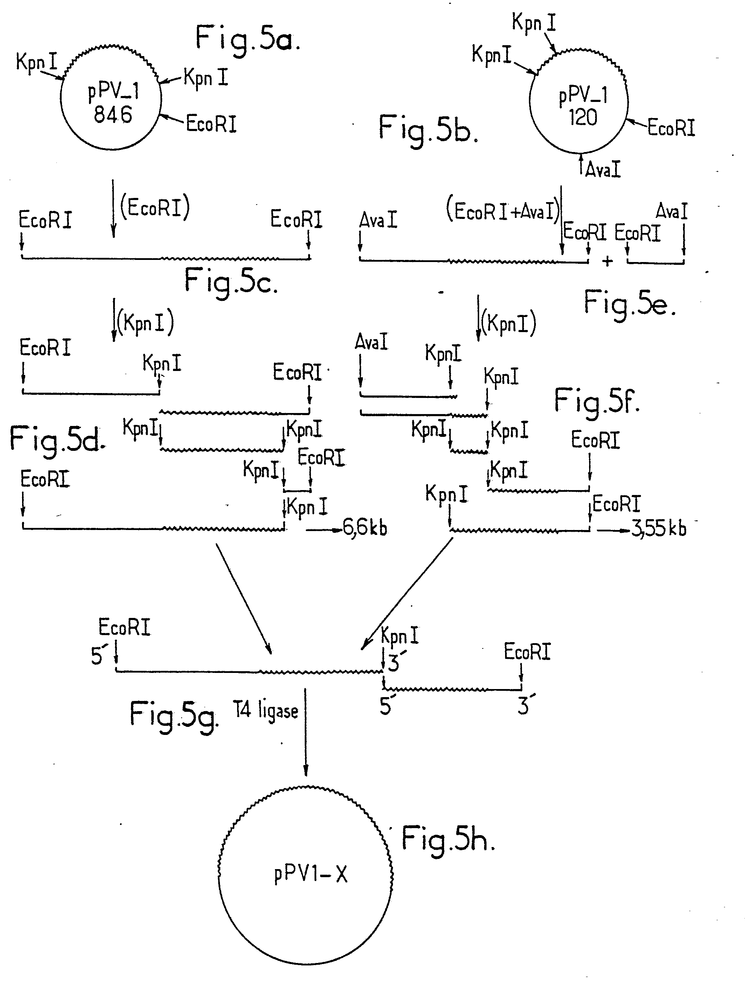

D'après les données de la figure 5, le génome entier comporte de 7 400 à 7 500 nucléotides, ce qui est en accord avec des déterminations antérieures.

Il apparaîtra donc clairement au spécialiste que la description qui précède définit la méthodologie à appliquer pour reproduire des essais de transformation de E.coli avec un vecteur, de préférence un plasmide, dans lequel aura auparavant été inséré un PV1-cADN-ARN hybride, pour isoler les clones contenant des fragments de cADN de PV1, et pour les analyser et repérer leurs emplacements relatifs vis à vis du génome entier initial dans la mesure où ils seraient différents des précédents.

Il est alors possible de reconstruire des cADN plus longs par recombinaison in vitro des fragments de cADN correspondants qui comportent des parties en chevauchement, en ayant recours aux enzymes de restriction communs appropriés, notàmment selon la technique dont un exemple est décrit ci-après

18

Exemple de production d'un cADN caractéristique, conforme
à l'invention.·
Obtention d'un plasmide portant l'extrémité 5' du cDNA
correspondant au génome du poliovirus PV1 et couvrant les
2/3 du génome viral

On procède à la recombinaison des deux plasmides des
clones pPVI-846 et pPVI-120.

On rappelle que :

- le clone pPVI-846 couvre la région du génome du poliovirus
qui s'étend du nucléotide 1($\pm$ 35) au nucléotide 3100 environ .
Il porte 2 sites de coupure par l'enzyme de restriction Kpn I
( positions  66 et 3050). Il n'a aucun site EcoRI (figure
5a) ;

- le clone pPVI-120 couvre la région du génome du poliovirus qui s'étend du nucléotide 2950 au nucléotide 5650
environ. Il porte 2 sites Kpn I (positions 3050 et 3 548.
Il n'a aucun site EcoRI ni AvaI (figure 5b ).

Le plasmide pBR322 dont sont dérivés les deux plasmides susdits, a dans son génome un site EcoRI (position 4359)
un site AvaI (1424), aucun site Kpn I.

Les plasmides et fragments d'ADN produits au cours
des opérations de recombinaison  sont représentés schématiquement dans les figures 5a    à  5h . Les parties représentées en zigzag sont originaires des cDNA correspondant
à des parties du poliovirus, celles en lignes pleines du
plasmide pBR 322 .

Procédure de recombinaison

1. Hydrolyse des DNA clonés par des enzymes de restriction

1.1. L'ADN du plasmide pPVI-846 est hydrolysé
complètement par EcoRI. La forme linéaire du DNA plasmidique ainsi obtenue (figure 5c) est hydrolysée par digestion partielle avec Kpn I; les fragments obtenus (figure 5d)
sont séparés par électrophorèse en gel d'agarose à 0.7 %.

Le fragment de taille 6.6 kbp est sélectionné.
Il représente en effet la séquence du plasmide pBR322 du
site EcoRI au site Pst I, prolongée de celle du DNA correspondant à la séquence du poliovirus qui s'étend du nucléotide 1 $\pm$ 35) au nucléotide 3050 (2e site Kpn I).

1.2. L'ADN du clone pPVI-120 est hydrolysé dans une digestion complète par AvaI et EcoRI formant ainsi 2 fragments de tailles différentes (figure 5e ). L'ADN est hydrolysé ensuite partiellement par Kpn I. Les fragments ainsi obtenus (figure 5f) sont séparés par électrophorèse en gel d'agarose à 0,7 %.

Le fragment de taille 3.55 kbp est sélectionné. Il représente en effet la séquence du cDNA du poliovirus allant du nucléotide 3050 (2e site Kpn I) au nucléotide 5650 environ, prolongée de celle des 752 paires de bases du segment Pst I-EcoRI du plasmide pBR322.

2. Extraction des fragments de DNA des gels

2.1. Les fragments sont visualisés dans les gels par coloration au bromure d'éthidium; ceux de la taille voulue sont extraits des gels par électroélution en sac à dialyse.

2.2. Le matériel ainsi obtenu est purifié et concentré.

3. Recollage des fragments (recombinaison)

Les deux fragments sélectionnés provenant des clones pPVI-846 et pPVI-120 et décrits ci-dessus sont mélangés et recollés à l'aide de la DNA ligase du phage T4 . Les bouts collants formés aux points de coupure par EcoRI et Kpn et portés à chaque extrémité des deux fragments favorisent leur recollage et assurent que celui-ci ne puisse se faire que dans le sens désiré (figures 5g et 5h).

Le génome du plasmide pBR322 est ainsi reconstitué sans modification ni délétion dans le plasmide recombinant. En particulier, les régions nécessaires à sa réplication et à l'expression de la résistance à la tétracycline ne sont pas touchées.

4. Transformation de la souche E.coli 1106

Les fragments des plasmides pPVI-846 et -120 recollés par leurs sites Kpn I et EcoRI sont mises en contact avec des bactéries compétentes de souche E.coli 1106 dans les conditions de transformation. Les colonies de bactéries résistantes à la tétracycline et sensibles à l'ampicilline sont sélectionnées.

5. <u>Analyse des nouveaux clones</u>

5.1 Le DNA plasmidique des bactéries tétracycline résistantes est purifié. Sa masse est déterminée par électrophorèse en gel d'agarose. Elle est égale à celle du plasmide pBR322 augmentée des 5650 paires de bases du cDNA viral formé par recombinaison.

5.2 L'hybridation in vitro des cDNA ainsi obtenus avec des sondes spécifiques provenant des clones pPVI-846 et pPVI-120 permet la vérification de la présence dans un seul clone recombinant du matériel génétique du poliovirus inséré originellement dans les deux clones parentaux.

5.3 L'analyse détaillée des nouveaux clones est effectuée par les méthodes utilisées antérieurement pour l'étude des clones déjà caractérisés (cartographie physique par enzymes de restriction, microscopie électronique, séquence, pouvoir pathogène, etc.).

5.4 Le cDNA porté par le plasmide recombinant (pPV1-X) porte l'information génétique nécessaire à la synthèse de la protéine p1, précurseur des protéines de capside VP4 (nucléotides 741 à 975), VP2 (nucléotides 976 à 1818), VP3 (1819 à 2562) et VP1 (2563 à 3501), suivie de celles qui correspondent à la protéine p2 (précurseur notamment de la protéine NCVPX) et au début de la protéine p3. L'ensemble couvre environ 5650 des 7432 bases du génome viral.

Le plasmide pPVI-846 a été déposé à la C.N.C.M. sous le n° I-155 et le plasmide 120 sous le n° I-156 le 19 mai 1981.

L'invention s'étend bien entendu à tous fragments d'ADN équivalents, notamment ceux dérivés des poliovirus appartenant à d'autres sérotypes, tels que ceux désignés sous les abréviations PV2 et PV3, et dépourvus des parties de gène codant pour les enzymes intervenant dans la réplication de ces virus. Les méthodes de fabrication des fragments de gène dérivés de PV1 s'appliquent de la même façon à l'obtention d'ADN correspondants de PV2 et PV3.

L'invention concerne également les fragments plus petits, notamment tels que ceux codant pour la protéine p1 (voir partie P1 sur la figure 4), notamment ceux s'étendant jusqu'à de 3 à 4 kilobases de l'extrémité 5' du génome du poliovirus. Ils sont utiles entre autres en tant que produits intermédiaires pour la fabrication des fragments plus grands décrits ci-dessus. Ils sont également intéressants par les produits d'expression dont ils dirigent la fabrication dans des bactéries compétentes.

22

## REFERENCES

1. Kitamura, N., Adler, C., and Wimmer, E. 1980. Ann. N.Y. Acad. Sci. 343-

3. Rueckert, R.R., Matthews, T.J., Kew, O.M., Pallansch, M., McLean, C., and Omilianowski, D. 1979. In : The Molecular Biology of Picornaviruses. R. Perez-Bercoff ed., pp. 113-125, Plenum, New York.

4. Flanegan, J.B., and Baltimore, D. 1979. J. Virol. 29, 352-360.

5. Etchison, D., and Ehrenfeld, E. 1980. Virology 107, 135-143.

6. Lee, Y.F., Kitamura, N., Nomoto, A., and Wimmer, E. 1979, J. Gen. Virol. 44, 311-322.

7. Kitamura, N., and Wimmer, E. 1980. Proc. Natl. Acad. Sci. USA 77, 3196-3200.

8. Nelson, T., and Brutlaq, D. 1979, Methods in Enzymol. 68, 41-50.

9. Wood, K.O. and Lee, J.C. 1976. Nuc. Ac. Res. 3, 1961-1971.

10. Zain, S., Sambrook, J., Roberts, J.J., Keller, W., Fried, M., and Dunn, A. 1979. Cell, 16, 851-861.

11. Murray, N.E., Brammer, W.J., and Murray, K. 1976. Mol. Gen. Genet. 150, 53-61.

12. Villa-Komaroff, L., Efstratiatis, A., Broome, S., Lomedico, P., Tizard, R., Naber, S.P., Chick, W.L., and Gilbert, W. 1978. Proc. Natl. Acad. Sci. USA 75, 3727-3731.

13. Grunstein, M., and Hogness, D. 1975. Proc. Natl. Acad. Sci. USA 72, 3961-3965.

14. Cami, B., and Kourilsky, P. 1978. Nuc. Acid. Res. 5, 2381-2390.

15. Denhardt, D.T. 1966. Biochem. Biophys. Res. Commun. 23, 641-646.

16. Clewel, D.B., and Helinski, D.R. 1970, Biochemistry 9, 4428-4439.

17. Birnboim, H.C., and Doly, J. 1979, Nuc. Acid. Res. 7, 1513-1522.

18. May, E., Kopecka, H. and May, P. 1975. Nuc. Acid. Res. 2, 1995-2005.

19. Maxam, A.M., and Gilbert, W. 1980. Methods in Enzymology, 65, 499-560.

20. Sanger, F., and Coulson, A.R. 1978. FEBS Letters, 87, 107-110.

21. Nomoto, A., Jacobson, A., Lee, Y.F., Dunn, J.J., and Wimmer, E. 1979. J. Mol. Biol. 128, 179-196.

22. Stewart, M.L., Crouch, R.J., and Maizel, J.V. 1980. Virology 104, 375-397.

23. Larsen, G., Semler, B., and Wimmer, E. 1981. J. Virol., 37, 328-335.

24. Hewlett, M.J. and Florkiewicz, R.Z. 1980. Proc. Natl. Acad. Sci. USA 77, 303-307.

25. Kupper, H., Keller, W., Kurz, C., Forss, S., Schaller, H., Franze, R., Strohmaier, K., Marquardt, O., Zaslavsky, V.G., and Hofschneider, P.H. 1981. Nature 289, 555-559.

26. Davis, R.W., Simon, M., and Davidson, N. 1971. Methods in Enzymology, 12, part D, 412-428.

## REVENDICATIONS

1 - ADN caractérisé en ce qu'il consiste ou est formé d'un segment hybridable à une partie du poliovirus du type PV-1, ce segment codant pour les protéines de capside et pour les éventuelles protéases nécessaires au découpage de celles-ci, cependant essentiellement à l'exclusion d'un fragment hybridable avec la partie terminale du poliovirus, comportant au moins 1,7 à 1,8 kilobases à partir de son extrémité terminale 3' et codant pour les protéines intervenant dans la réplication du poliovirus.

2 - ADN selon la revendication 1, caractérisé en ce qu'il comprend, du côté de son extrémité 5', les nucléotides d'extrémité du poliovirus du type PV-1, plus particulièrement la séquence

```
                    10          20          30          40
(G)20 TTAAAACAGC TCTGGGGTTG TACCCACCCC AGAGGCCCAC
                    50          60          ·70          80
      GTGGCGGCTA GTACTCCGGT ATTGCGGTAC CCTTGTACGC
                    90         100
      · CTGTTTTATA CTCCCTTCCC
```

3 - ADN selon la revendication 1 ou la revendication 2, caractérisé par une série successive de sites de restriction dont les positions vis-à-vis d'un site Bam HI de référence, lui-même situé à une distance de l'ordre de 0,7 kb de l'extrémité 5' du génome du poliovirus sont les suivantes (distances exprimées en kb et affectées d'un signe moins "-", pour ceux des sites situés entre ladite extrémité 5' du génome du poliovirus et du site BamHI considéré) :

```
Pst I   : 1,1   ; 1,55 ; 2,75
Pvu II  : 2,6
Kpn I   : -0,63 ; 2,77 ; 3,37
Bgl II  : 4,9
Bam HI  : -0,45 ; 1,4  ; 3,9
Bal I   : -0,05 ; 2,80 ; 3    ; 4,25
Bst EII :  2,55 ; 3,20
Bgl I   : -0,66 ; 4,56
Bcl I   :  2,50 ; 3,25
```

4 - ADN selon la revendication 3, caractérisé par une extrémité 5' formée, soit par le site Bam HI de référence, lorsque toute la partie antérieure a été délétée, soit par l'extrémité Bal I (-0,05 kb), ou Kpn I (-0,63 kb) ou Bgl I (-0,66 kb), notamment en cas de délétions partielles de ladite partie antérieure.

5 - ADN selon la revendication 4, caractérisé par une extrémité 3', soit Bgl II (4,9 kb), soit Bgl I (4,56 kb), soit Bal I (4,25 kb), soit Bam HI (3,9 kb).

6 - ADN selon l'une quelconque des revendications 1 à 5, caractérisé par sa capacité d'hybridation avec les sondes d'oligonucléotides dérivés de l'ARN du poliovirus PV-1 et résistant à la nucléase T1 et comportant des extrémités nucléotidiques 5', dont les positions respectives dans la séquence nucléotidique totale de l'ARN du poliovirus (repérées en nombres (N) de paires de bases comptées à partir de sa propre extrémité 5') sont les suivantes :

N806  (oligonucléotide n° 2)
N3000 (oligonucléotide n° 1)
N4434 (oligonucléotide n° 9).

7 - ADN selon l'une quelconque des revendications 1 à 6, caractérisé en ce qu'il est incorporé à un vecteur, plus particulièrement plasmide, notamment du type pBR 322.

8 - Procédé de fabrication d'un ADN conforme à la revendication 1, caractérisé en ce qu'il comprend une opération d'hydrolyse totale ou partielle selon le cas d'un cADN correspondant au poliovirus PV-1, sous l'action d'une enzyme de restriction Bgl II, Bgl I, Bal I ou Bam HI et la récupération de celui des fragments qui est hybridable avec les susdits oligonucléotides n° 2, 1 et 9.

9 - Procédé selon la revendication 8, caractérisé en ce qu'il comprend en outre, s'il en est besoin, notamment en vue de la fabrication d'un ADN conforme à la revendication 2 ou 3, le clonage dans une cellule hôte appropriée d'un cADN-ARN hybride comprenant une extrémité 5' correspondant à l'extrémité 5' du poliovirus PV-1, la récupération de deux des clones qui contiennent alors un cADN comportant ladite extrémité, la détermination des zones communes ou de recouvrement de ce dernier cADN et du cADN précédemment obtenu, la réalisation d'une recombinaison génétique entre ces deux cADN, notamment par la mise en jeu d'une des enzymes de restriction auxquelles correspondent, dans lesdites zones de recouvrement, des sites de restriction communs.

10 - Application d'un ADN conforme à l'une quelconque des revendications 1 à 7 à la production de principes antigéniques, comprenant la transformation avec cet ADN d'une cellule hôte dans laquelle il est susceptible d'être exprimé, le cas échéant après incorporation préalable de cet ADN dans un vecteur approprié, contenant un promoteur susceptible d'initier la traduction de cet ADN dans ladite cellule hôte, et la récupération à partir des cellules transformées ou de leur milieu de culture, selon le cas, des principes antigéniques produits.

11 - Micro-organisme, tel que bactérie *E.coli*, hébergeant un ADN conforme à l'une quelconque des revendications 1 à 7.

Fig.1.

Fig.2.

Fig.3.

Kilobases

1    2    3    4    5    6    7

P1 ──────────▶  P2 ──────▶  P3 ──────▶

| Enzyme | | | | | | | |
|---|---|---|---|---|---|---|---|
| Pst I | 1,8 | 0,45 | 1,2 | 4,0 | | | |
| Hind III | 6.1 | | | | 0,45 | 0,93 | |
| Pvu II | 3,3 | | 3,8 | | | 0,39 | |
| Kpn I | 0,07 | 3,00 | 0,60 | 3,80 | | | |
| Bgl II | 5,6 | | | | 1,85 | | |
| BamHI | 0,25 0,45 | 1,4 | 2,5 | 2,9 | | | |
| Bal I | 0,65 | 2,85 | 0,20 | 1,25 | 1,30 | 1,25 | |
| BstE II | 3,25 | 0,65 | 3,6 | | | | |
| Bgl I | 0,04 | 5,3 | 2,15 | | | | |
| Bcl I | 3,20 | 0,75 | 3,55 | | | | |

846

818

366

146

120

751

341

378

Fig.4.

0065924

Fig.5a.

Fig.5b.

Fig.5c.

Fig.5d.

Fig.5e.

Fig.5f.

Fig.5g.

Fig.5h.

Office européen
des brevets

**RAPPORT DE RECHERCHE EUROPEENNE**

Numéro de la demande

EP 82 40 0941

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| P,X | PROC. NATL. ACAD. SCI., vol. 78, no. 10, octobre 1981, pages 5983-5987, (USA); S.VAN DER WERF et al.: "Molecular cloning of the genome of poliovirus type 1". *En entier* | 1-11 | C 12 N 15/00 C 07 H 21/04 C 12 N 1/20 C 12 P 21/02 // C 12 R 1/19 A 61 K 39/13 |
| | --- | | |
| P,X | PROC. NATL. ACAD. SCI., vol. 78, no. 8, août 1981, pages 4887-4891, (USA); V.R.RACANIELLO et al.: "Molecular cloning of poliovirus cDNA and determination of the complete nucleotide sequence of the viral genome". *En entier* | 1,7,10 ,11 | |
| | --- | | |
| A | PROC. NATL. ACAD. SCI., vol. 73, no. 7, juillet 1976, pages 2191-2195, (USA); D.L.KACIAN et al.: "Synthesis of extensive, possibly complete, DNA copies of poliovirus RNA in high yields and at high specific activities". *En entier* | 1 | DOMAINES TECHNIQUES RECHERCHES (Int. Cl. ³) C 12 N C 12 P C 12 R C 07 H A 61 K |
| | --- | | |
| A | ANNALS OF THE NEW-YORK ACAD. OF SCIENCES, vol. 354, 1980, pages 183-201; N.KITAMURA et al.: "Structure and expression of the picornavirus genome". | | |
| | --- | | |
| | -/- | | |

Le présent rapport de recherche a été établi pour toutes les revendications

| Lieu de la recherche | Date d'achèvement de la recherche | Examinateur |
|---|---|---|
| LA HAYE | 18-08-1982 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.......................................................................

& : membre de la même famille, document correspondant

OEB Form 1503. 03.82

Office européen
des brevets

**RAPPORT DE RECHERCHE EUROPEENNE**

Numéro de la demande

EP  82 40 0941

| | DOCUMENTS CONSIDERES COMME PERTINENTS | | Page  2 |
|---|---|---|---|

| Catégorie | Citation du document avec indication, en cas de besoin, des parties pertinentes | Revendication concernée | CLASSEMENT DE LA DEMANDE (Int. Cl. ³) |
|---|---|---|---|
| A | PROC. NATL. ACAD. SCI., vol. 77, no. 6, juin 1980, pages 3196-3200, (USA); N.KITAMURA et al.: "Sequence of 1060 3'-terminal nucleotides of poliovirus RNA as determined by a modification of the dideoxynucleotide method". | | |
| | ----- | | |
| | | | **DOMAINES TECHNIQUES RECHERCHES (Int. Cl. ³)** |

Le présent rapport de recherche a été établi pour toutes les revendications

| Lieu de la recherche | Date d'achèvement de la recherche | Examinateur |
|---|---|---|
| LA HAYE | 18-08-1982 | DESCAMPS J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................

& : membre de la même famille, document correspondant

OEB Form 1503 03 82